# EUROPEAN PATENT SPECIFICATION

(11) **EP 2 098 211 B1**
(45) Date of publication and mention of the grant of the patent: **18.05.2011**
(21) Application number: 09154288.6
(22) Date of filing: 04.03.2009
(51) Int. Cl.: A61H 23/04

(54) **Compression foot cuff**
Kompressionsfußmanschette
Collier de pied de compression

(30) Priority: 04.03.2008 US 41947
(43) Date of publication of application: 09.09.2009
(62) Divisional of application: 09170796.8
(73) Proprietor: Tyco Healthcare Group LP, Mansfield, MA 02048 (US)
(72) Inventor: Nardi, Steven, Taunton, MA 02780 (US)
(74) Representative: Körber, Martin Hans

(56) References cited:
- US-A- 4 805 601
- US-A- 5 931 797
- US-A1- 2005 143 682
- US-A1- 2007 282 233

## Description

### FIELD OF THE INVENTION

The present invention relates to a compression foot cuff as claimed in claim 1 and to a method of making a compression foot cuff as claimed in claim 11.

### BACKGROUND

Compression devices for applying compressive forces to a selected area of a wearer's anatomy are generally employed to improve blood flow in the selected area. Compression devices that provide intermittent pulses of a compressed fluid (i.e. air) to inflate at least one inflatable chamber in a cuff or sleeve are particularly useful. This cyclic application of pressure provides a non-invasive method of prophylaxis to reduce the incidence of deep vein thrombosis (DVT), and the like. These compression devices find particular use during surgery on patients with high-risk conditions such as obesity, advanced age, malignancy, or prior thromboembolism. Patients who develop this condition often have swelling (edema) and tissue breakdown (venous stasis ulcer) in the lower leg. When a DVT occurs, the valves that are located within the veins of the leg can be damaged, which in turn can cause stasis and high pressure in the veins of the lower leg.

Generally, these compression devices are fluidly coupled to a source of pressurized fluid by one or more air tubes. Additionally, each compression device includes a flexible shell having one or more bladders disposed therein. The compression device is placed around the patient's foot or other selected portion whereupon a pressurized fluid is delivered into the bladder creating pressure at the part or parts of the body in contact with the bladder.

Compression cuffs adapted for use with a patient's foot may be used by themselves or combined with one or more additional compression cuffs or sleeves that are disposed on portions of a patient's leg for improving the treatment regimen. In general, each of the additional compression sleeves includes a plurality of separate inflatable chambers that are progressively arranged along a longitudinal axis of the sleeve from a lower portion to an upper portion of the limb. A pressure source, e.g. a controller, is provided for intermittently forming a pressure pulse within these inflatable chambers from a source of pressurized fluid during periodic compression cycles. The compression sleeves provide a pressure gradient along the patient's limbs during these compression cycles which progressively decreases from the lower portion to the upper portion of the limb (e.g. from the ankle to the thigh).

Compression cuffs that are adapted for use with a patient's foot generally include a heel strap with a tab portion that is adapted to fit around a portion of the patient's heel. This arrangement allows the compression cuff to be wrapped around and releasably attached to the patient's foot. The compression cuff may include a generally planar, rigid sole to direct expansion of the inflatable chamber toward the wearer's foot. The rigid sole needs to be located under that portion of the inflatable member that is acting on the portion of the foot to produce blood flow out of the foot. Applicants have discovered that the conventional planar, rigid sole may be uncomfortable for the wearer because of the rigidity of the sole.

Examples of compression cuffs are disclosed in U.S. Pat. Nos. 4,013,069 and 4,030,488 to Hasty, U.S. Pat. Nos. 4,029,087 and 5,795,312 to Dye, U.S. Pat. No. 5,626,556 to Tobler et al., and US-A1-2007/02822 33 to Meyer et al., all of which are currently owned by Tyco Healthcare Group LP. Other examples of compression cuffs are disclosed in U.S. Patent Nos. 4,696,289 to Gardner et al., 5,989,204 to Lina and 5,345,260 to Cook. An example of compression treatment method is disclosed in U.S. Pat. No. 6,231,532 to Watson et al., which is owned by Tyco Healthcare Group LP.

Further examples of compression cuffs having an inflatable member defining an inflatable chamber and having a sole secured to the foot cuff are disclosed in U.S. pat. No. 4,805,601, in U.S. pat. No. 5,931,797 and further in U.S. patent application US 2005/0143682 A1.

U.S. patent application US 2007/028223 A1, which forms the preamble of independent claim 1, discloses a compression foot cuff with an inflatable member defining an inflatable chamber and a sole secured to the foot cuff. The sole is hereby provided in generally opposing relationship with the first layer of the inflatable member.

### SUMMARY

According to the present invention a compression foot cuff for applying compression to a wearer's foot comprises an inflatable member including first and second fluid impermeable layers secured to one another to define an inflatable chamber. A sole is secured to the foot cuff in generally opposing relationship with the first layer of the inflatable member. The sole includes a biasing member, biasing the sole out-of-plane along axes perpendicular to its major axis in a direction causing a central area of the sole to move toward the inflatable member and longitudinal ends of the sole to move away from the inflatable member.

The present invention further relates to a method of making a compression foot cuff for applying compression to a wearer's foot. The method comprises the step of forming an inflatable member by joining together generally opposed first and second layers of fluid impermeable material. A sole is formed including a biasing member, biasing a sole out-of-plane along axes perpendicular to its major axis. The sole is secured to the foot cuff in generally opposing relationship with the first layer of the inflatable member, so that the biasing member biases the sole in a direction causing a central area of the sole to move towards the inflatable member and longitudinal ends of the sole to move away from the inflatable member.

Other features will be in part apparent and in part pointed out hereinafter.

### BRIEF DESCRIPTION OF THE DRAWINGS

FIG. 1 is an exploded perspective of an example of a compression foot cuff;

FIG. 2 is a perspective of a bladder of the foot cuff with a sole of a first example attached thereto;

FIG. 3 is a perspective of the sole in FIG. 2;

FIG. 4 is a front elevation of the sole in FIG. 3;

FIG. 5 is similar to FIG. 4 with the sole bent in a concave configuration;

FIG. 6 is similar to FIG. 4 with the sole bent in a convex configuration;

FIG. 7 is a perspective of a bladder of a foot cuff with a sole of a second example attached thereto;

FIG. 8 is a perspective of the sole in FIG. 7;

FIG. 9 is a front elevation of the sole in FIG. 8;

FIG. 10 is similar to FIG. 9 with the sole bent in a convex configuration;

FIG. 11 is a perspective of a sole of a first embodiment;

FIG. 12 is a front elevation of the sole in FIG. 11;

FIG. 13 is a perspective of a sole of a second embodiment; and

FIG. 14 is a front elevation of the sole in FIG. 13.
Whereas Figs. 11-14 show soles of embodiments of the present invention, the other Figs. show examples useful for understanding the invention.

Corresponding reference characters indicate corresponding parts throughout the drawings.

### DETAILED DESCRIPTION OF THE DRAWINGS

Referring now to the drawings, a compression foot cuff for applying compressive pressure to a wearer's foot is generally indicated at 10. The foot cuff is adapted for use in a compression therapy system, which further includes a supply of pressurized air (not shown) and tubing connecting the supply of pressurized air to the foot cuff.

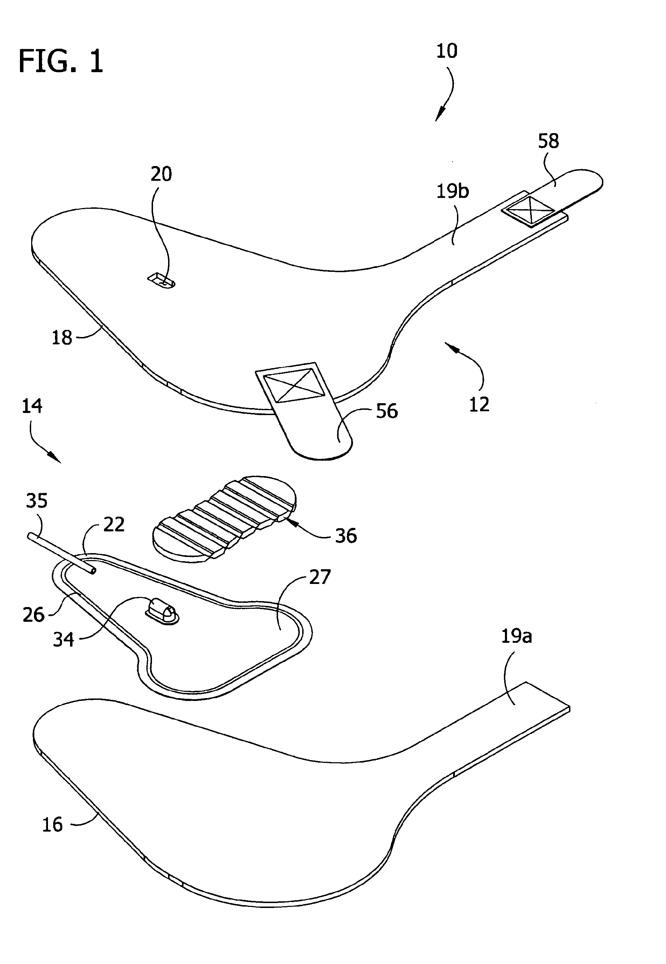

As shown best in FIG. 1, the foot cuff 10 includes an envelope, generally indicated at 12, substantially enveloping or enclosing a bladder, generally indicated at 14. The envelope 12 includes an inner contact layer 16 and an outer layer 18 secured to one another generally adjacent to corresponding perimeters of the layers to define an interior space for receiving and substantially enclosing the bladder 14 (broadly, "an inflatable member") therein. The contact layer 16 and the outer layer 18 may be fixedly secured to one another at their peripheral edge margins, such as by heat welding, adhesives, sewing or other suitable ways. Alternatively, the contact layer 16 and the outer layer 18 may be releasably secured to one another. In use the contact layer 16 is adjacent to the wearer's foot and the outer layer 18 is located farthest from the foot. As used herein, the terms "inner" and "outer" indicate relative positions of respective components and surfaces with respect to the skin of the wearer's body part when the compression device is secured to the body part, and as such, an "inner" component or surface is more adjacent to the skin of the body part than an "outer" component or surface.

Contact layer 16 and outer layer 18 of the envelope 12 include ankle strap portions 19a and 19b respectively. Ankle strap portions 19a, 19b have a longitudinally projecting configuration for wrapping about a portion of the foot adjacent to the ankle. The ankle strap portions 19a, 19b can be sewn, RF welded, or sonic welded. However, in the illustrated embodiments, the ankle strap portions 19a, 19b are formed as one piece with the contact layer 16 and outer layer 18, respectively.

Contact layer 16 of the envelope 12 is adapted for contacting the foot. Contact layer 16 is in one embodiment fabricated from a chemically treated material, with wicking ability, for wicking away moisture from the skin. In one example, contact layer 16 includes a mesh-like fabric capable of wicking moisture away from the patient's skin. Furthermore, the contact layer 16 can be faced with a soft material toward the treatment surface of the patient. For example, the material can be a thin layer of open celled porous foam, napped cloth, or a layer of vapor permeable cloth permeable. It is understood that the cuff 12 may not include a contact layer.

Outer layer 18 of the envelope 12 includes an opening 20 for permitting a pressurized fluid inlet passage therethrough. Outer layer 18 is configured for providing the attachment surface for a hook and loop feature of cuff 12, as will be described in more detail herein below. Moreover, the outer layer 18 provides a soft material for cushioning effect against the top portion of the feet and may be fabricated from similar materials as contact layer 16 and in similar dimensions therewith for corresponding geometry. Alternatively, outer layer 18 may be fabricated from a laminated material, such as, for example, sontara fabric, open cell urethane foam, or loop fabric. It is understood that the cuff 12 may not include an outer layer.

The bladder 14 is configured for positioning against the bottom portion of the foot. The bladder 14 includes an outer layer 22 and an inner layer (not shown) of air impermeable material (e.g., PVC) joined together in a suitable manner along a line 26 adjacent to their peripheries to define a single inflatable chamber 27. The layers 22 may be joined to one another in a suitable manner such as by radio frequency (RF) welding. Other ways of joining the layers 22 include sewing, adhesive, heat sealing, etc. It is understood that the bladder 14 can include more than one inflatable chamber 27. The inflatable chamber 27 of the bladder 14 is adapted for receiving and retaining a pressurized fluid (e.g. air) for exerting compressive pressure to the foot during successive pressure application cycles. The inflatable chamber 27 has a port 34 and a tube 35 connected to the port for air or fluid to be introduced into the chamber during the start of a compression cycle and to be exhausted to end the compression cycle. The port 34 of the illustrated example is a plastic component that is secured such as by heat welding or other means to the bladder 14. It is understood that other ways of introducing air or fluid into the chamber 27 are possible.

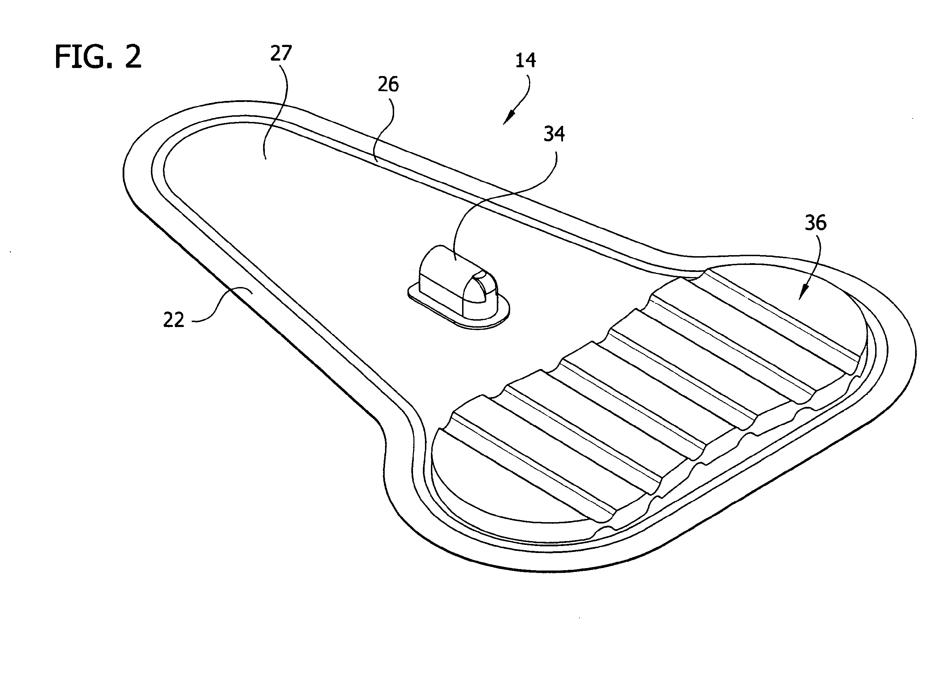

Referring still to FIGS. 1-6, a sole, generally indicated at 36, is disposed between the outer layer 18 of the envelope 12 and the outer layer 22 of the bladder 14 and is positioned to underlie the foot in use. The sole 36 may be secured to the outer layer 22 of the bladder or to the outer layer 18 of the envelope 12 using adhesive, for example, or the sole may be secured in position in other ways. Generally, the sole 36 is bendable out-of-plane to provide flexibility and increased comfort to the patient. It is believed the sole 36 provides a counterforce to the outer layer 22 of the bladder 14 as the bladder is expanding to direct expansion toward the contact layer 16 and the user's foot. In this way, the inner layer 24 expands outward more than the outer layer 22 to direct compressive force toward the user's foot.

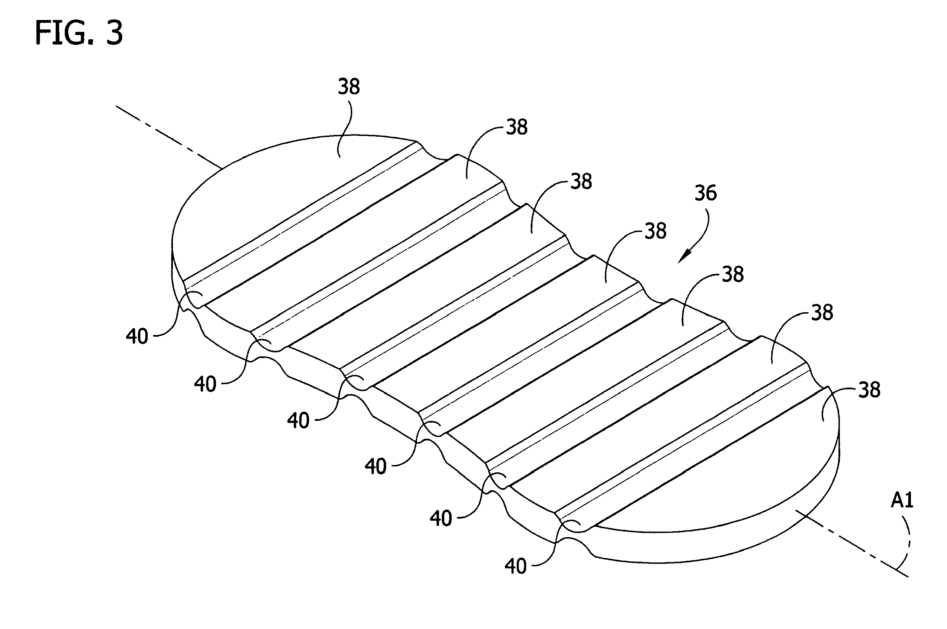

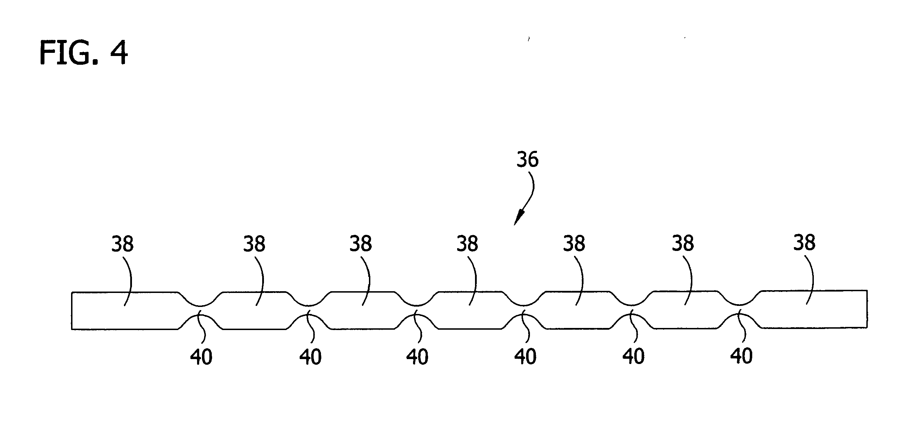

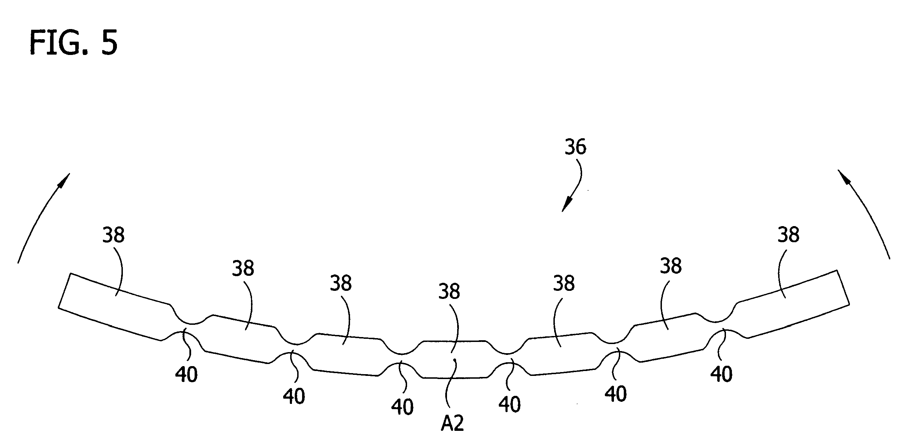

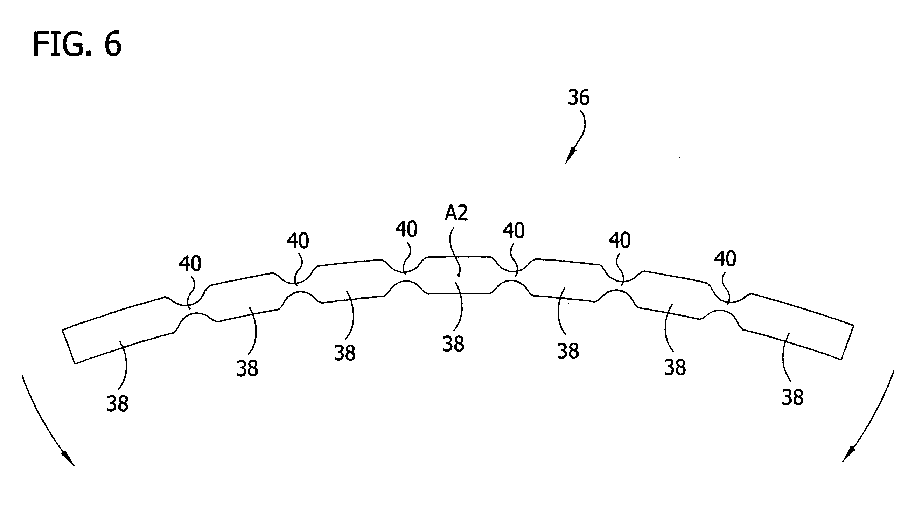

The sole 36 includes a plurality of generally rigid members 38 that are spaced apart along a major axis A1 (FIG. 3) of the sole extending between opposite ends. The generally rigid members 38 themselves are substantially unbendable in-plane and out-of-plane. The generally rigid members are hingedly secured to one another along the major axis A1 by generally flexible members 40. The generally flexible members 40 act as living hinges to allow the sole 36 as a whole to bend out-of-plane. More specifically and referring to FIGS. 5 and 6, the opposite axial ends of the sole 36 are bendable or flexible about a central transverse axis A2 lying in a plane generally transverse to the major axis A1. The axial ends of the sole 36 may be flexed either inward (FIG. 5) or outward (FIG. 6) about the central transverse axis A2 so that the sole may be bent in a generally concave configuration (FIG. 5) or in a generally convex configuration (FIG. 6), respectively. In the illustrated example the sole 36 is formed as a one-piece, integral structure. The sole 36 of the illustrated example may be constructed from a polypropylene material using injection molding manufacturing techniques. As shown best in FIGS. 4-6, the generally flexible members 40 are thinner than the generally rigid members so that the generally flexible members function as living hinges. Other ways of making the sole 36 bendable out-of-plane, including other ways of making the generally rigid members 38 hingedly secured to one another, are possible. For example, the generally rigid members 38 may be formed separately and secured to one another by hinge pins or other devices to make a hinged connection.

Referring to FIG. 1, hook fasteners 56, 58 are provided for securing the wrapped cuff 12 around a foot, and are positioned on the outer layer 18 of the cuff. Hook fastener 56 is mounted to strap portion 19b of outer layer 18 of foot cuff 12 while hook fastener 58 is mounted on a surface of outer layer 18. In use, when ankle strap portions 19a, 19b are wrapped about the back of the foot, hook element 56 engages outer layer 18 to facilitate mounting of foot cuff 12 on the foot. An identification tab (not shown) may also be included for providing information such as the model number and manufacturer name. Hook fasteners 56, 58 may have tabs (not shown) without fastening material thereon to provide convenient gripping locations on the hook fasteners to thereby allow the practitioner to easily remove the hooks from the outer face 18b of outer layer 18. The use and operation of the foot cuff 12 for applying compression therapy to the wearer's foot is generally known in the art and will not be described herein.

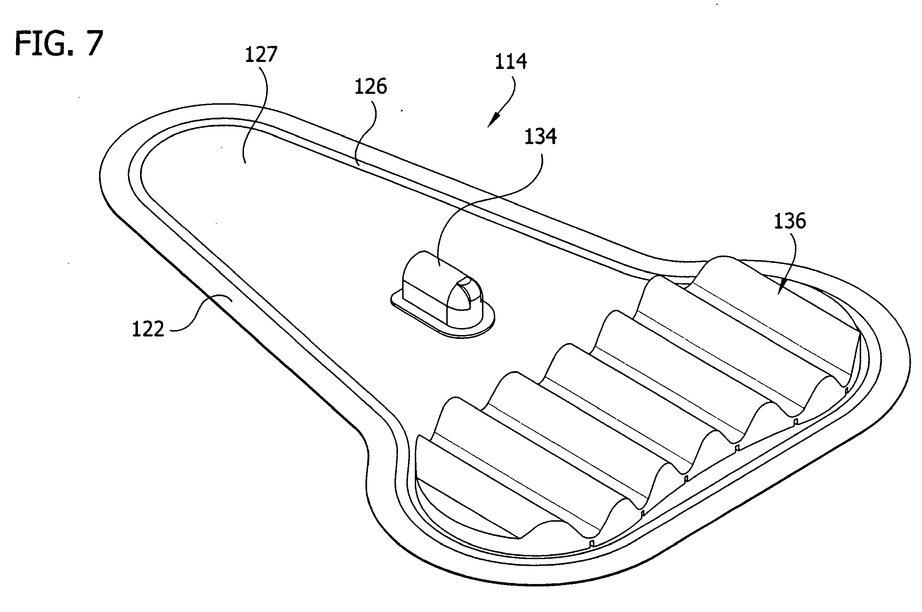

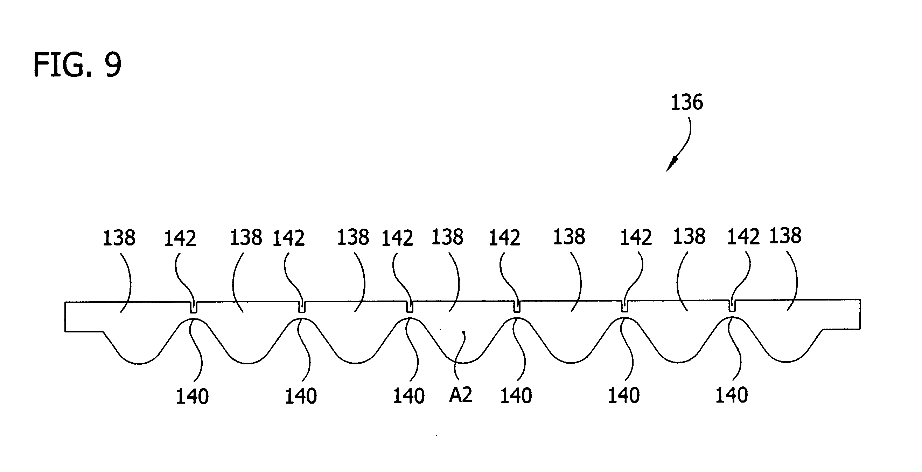

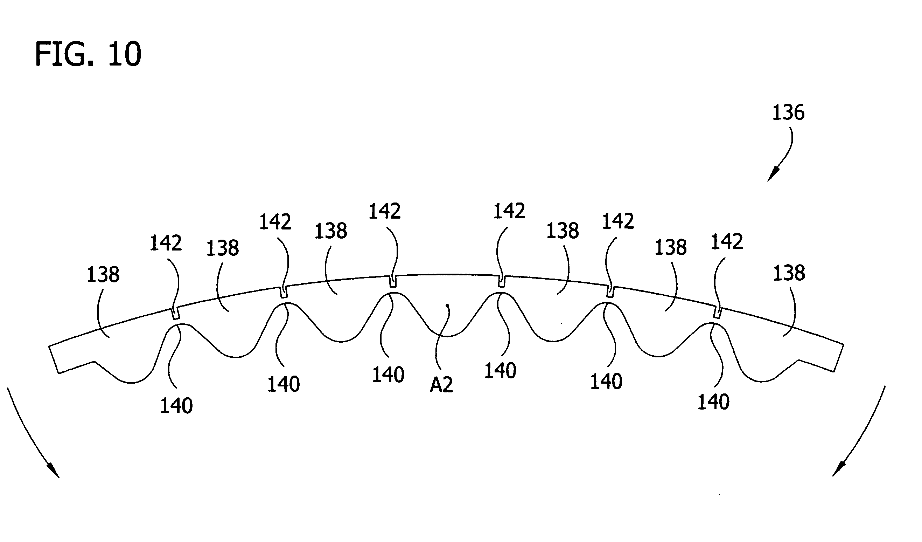

Referring now to FIGS. 7-10, a second example of a sole for a compression device is generally indicated at 136. This example is similar to the prior example, and therefore, like components are indicated by corresponding reference numerals, plus 100. The main difference between the present sole 136 and the prior example 36 is that the present sole is constructed to be bendable out-of-plane in one direction only. More specifically, the axial ends of the present sole 136 are bendable only outward about a central transverse axis A2 so that the sole is configurable in a convex configuration (FIG. 10). In other words, the sole 136 can bend in a direction that would cause the central portion of the sole to move in a direction toward the bladder 114 and the ends to move in a direction away from the bladder. This is not to say, the sole 136 moves relative to the bladder 114, but the bladder provides a reference frame for describing how the sole may bend. The sole 136 is generally not configurable in a concave configuration. Stated another way, the sole 136 resists bending in a direction causing the central portion of the sole to move away from the bladder 114 and the ends of the sole to move in a direction toward the bladder. In this way, the sole 136 is bendable to generally conform to the arch of a bottom of a foot while it is believed that the sole will provide a better counterforce to the outer layer 22 of the bladder 14 as the bladder is expanding to direct expansion toward the contact layer 16 and the user's foot.

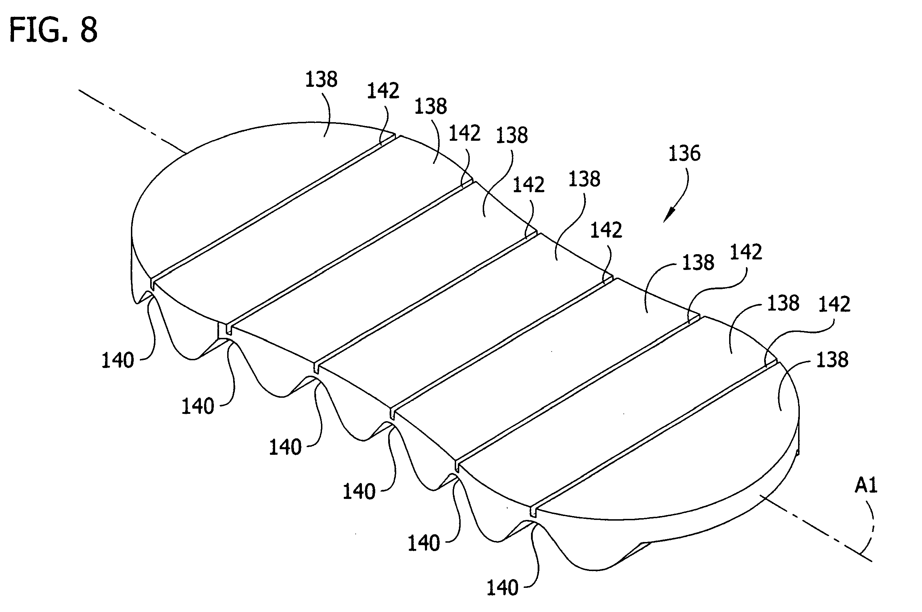

In the illustrated example, the sole 136 includes a plurality of generally rigid members 138 that are spaced apart along a major axis A1 of the sole extending between opposite ends. The generally rigid members 138 themselves are substantially not bendable in-plane or out-of-plane. The generally rigid members 138 extend generally transverse to the major axis A1 of the sole and have inner surfaces that are generally planar. The inner surfaces are in generally opposing relationship with the outer layer 22 of the bladder 14. The generally rigid members 138 taper away from the bladder 114 so that the members have generally triangular cross-sectional shapes. The generally rigid members are hingedly secured to one another along the major axis A1 by generally flexible members 140. Referring to FIGS. 8-10, transverse slots 142 in the flexible members 140 extending through the inner surface of the sole 136 allow the axial ends of the sole to flex or bend outward about a central transverse axis and prevent the ends from bending inward so that the sole as a whole is bendable only in the convex configuration. It will be understood that adjacent rigid members 138 will engage each other after only a small amount of bending toward a con cave configuration. Thus, bending in this direction is resisted. Other ways of making the sole capable of unidirectional, out-of-plane bending is possible. In the illustrated example, the sole 136 is formed as a one-piece, integral structure. The sole of the illustrated embodiment may be constructed from a polypropylene material using injection molding manufacturing techniques.

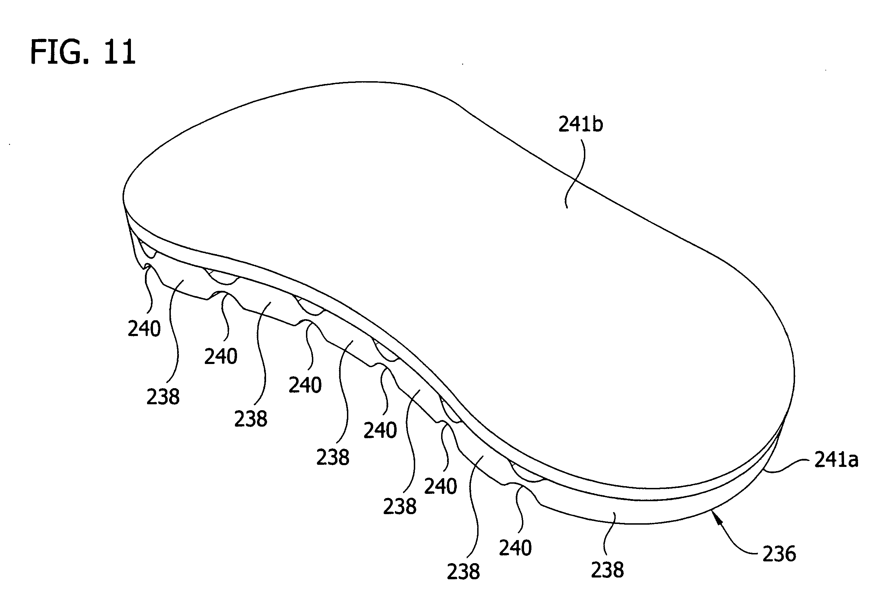

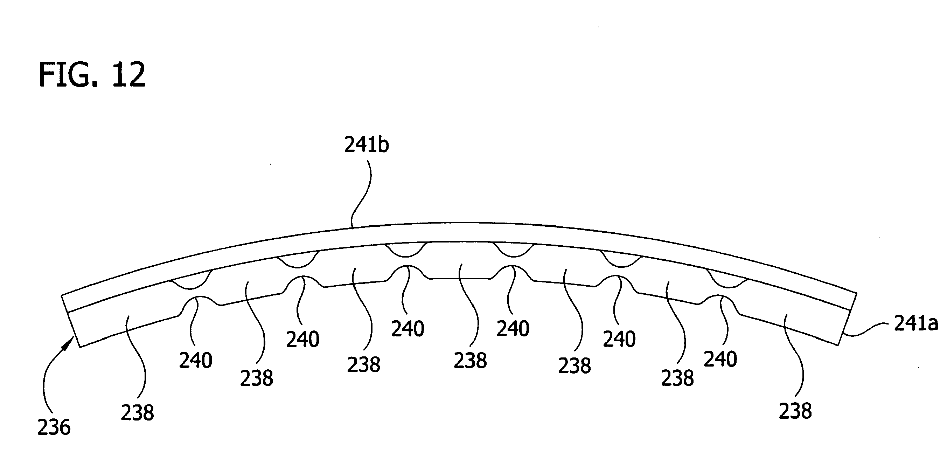

Referring to FIGS. 11 and 12, a first embodiment of a sole for a compression device is generally indicated at 236. This embodiment is similar to the first example, and therefore, like components are indicated by corresponding reference numerals, plus 200. The main difference between the sole 236 and the first example 36 is that the sole 236 comprises a flexible member 241a and a biasing member 241b. The biasing member 241b is secured to the sole 236 to bias it in the generally convex configuration. The flexible member 241 a can be made of a soft material and provide no resistance to bending in any direction. The biasing member 241b of the sole 236 provides resistance to bending to the concave configuration and thus supports the bladder to apply pressure to the foot, when the bladder is inflated. However, the biasing member 241b preferably provides less resistance to bending toward the concave configuration. In the illustrated embodiment, the biasing member 241b is secured to flexible member 241 b on the outer side of the sole 236 (i.e., adjacent to the outer layer 18 of the envelope 12), although the biasing member may be secured to the inner side of the sole within the scope of the invention as claimed. The biasing member 241b may be formed from a generally resilient material including plastic and metal. It is understood that the sole 236 may be of other configurations within the scope of the invention as claimed. Although the biasing member 241 b has been shown as a flat, uniform piece of material, it may have other configurations, including configurations that are not contiguous with the perimeter of the flexible member 241a.

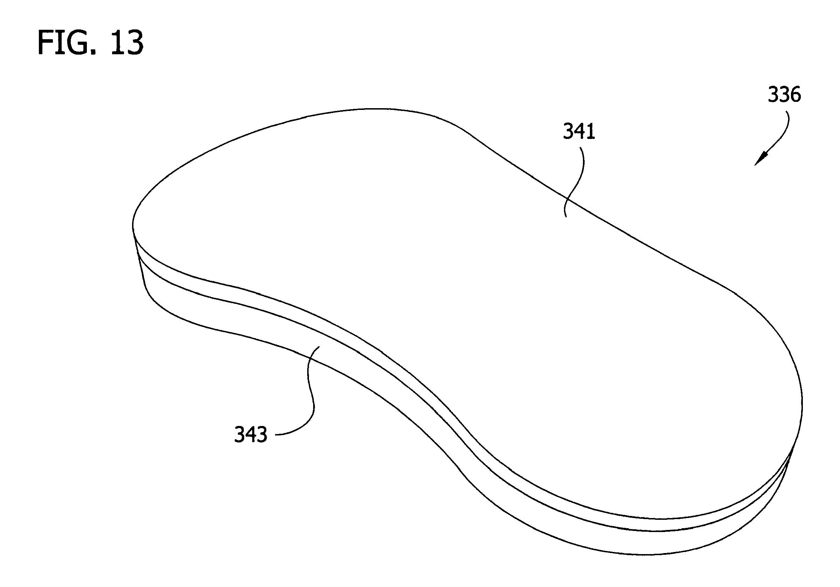

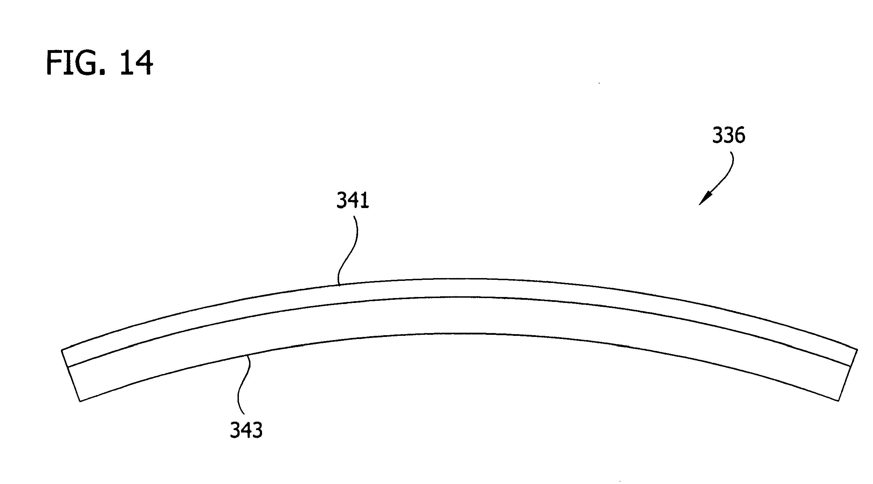

Referring to FIGS. 13 and 14, a second embodiment of a sole for a compression device is generally indicated at 336. This embodiment is similar to the first example, and therefore, like components are indicated by corresponding reference numerals, plus 300. The main difference between the present sole 336 and the first example 36 is that the sole of the present embodiment is a laminate including a compressible member 343 and a biasing member 341 secured to the compressible member to bias it in the generally convex configuration. In the illustrated embodiment, the biasing member 341 is secured to an outer side of the compressible member 343 (i.e., adjacent to the outer layer 18 of the envelope 12), although the biasing member may be secured to the inner side of the compressible member within the scope of the invention as claimed. The biasing member 341 may be formed from a generally resilient material including plastic and metal. The compressible member 343 may be formed from a spongy or generally compressible material. The compressible member 343 provides increased comfort to the wearer. Other materials and other configurations of the biasing member and the compressible member are within the scope of the present invention as claimed.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

## Claims

1. A compression foot cuff (10) for applying compression to a wearer's foot comprising:
an inflatable member(14) including first and second fluid impermeable layers (22, 24) secured to one another to define an inflatable chamber (27); and
a sole (236, 336) secured to the foot cuff (10) in generally opposing relationship with the first layer (22) of the inflatable member (14);
**characterized in**
**that** the sole (236, 336) includes a biasing member (241b, 341) biasing the sole (236, 336) out-of-plane along axes perpendicular to its major axis (A1) in a direction causing a central area of the sole (2236, 336) to move toward the inflatable member (14) and longitudinal ends of the sole (2236, 336) to move away from the inflatable member (14).

2. A compression foot cuff (10) as set forth in claim 1 wherein the sole (236) comprises a flexible member (241a) and a biasing member (241b) and the biasing member (241b) is secured to the flexible member (241a) to bias it in the generally convex configuration.

3. A compression foot cuff (10) as set forth in claim 2 wherein the biasing member (241b) is secured to an outer or an inner side of the flexible member (241a).

4. A compression foot cuff (10) as set forth in any of claims 2 to 3
wherein the flexible member (241a) includes a plurality of spaced apart, generally rigid members (238) hingedly secured to one another along the major axis (A2) of the sole (236) so that the sole (236) is generally bendable out-of-plane.

5. A compression foot cuff as set forth in claim 4 wherein the generally rigid members (238) are hingedly secured to one another by a generally flexible member (240).

6. A compression foot cuff as set forth in claim 5 wherein the generally rigid members (238) have thicknesses greater than the generally flexible member (240).

7. A compression foot cuff as set forth in claim 6 wherein the rigid members (238) and the generally flexible member (240) are formed as one piece of material.

8. A compression foot cuff (10) as set forth in one of the claims 5 to 7 comprising a plurality of generally flexible members (140).

9. A compression foot cuff (10) as set forth in claim 1 wherein the sole (336) is a laminate including a compressible member (343) and the biasing member (336) is secured to the compressible member (343) to bias it in the generally convex configuration.

10. A compression foot cuff (10) as set forth in claim 9 wherein the biasing member (336) is secured to an outer or an inner side of the compressible member (343).

11. A method of making a compression foot cuff (10) for applying compression to a wearer's foot, the method comprising:
forming an inflatable member (14) by joining together generally opposed first and second layers (22, 24) of fluid impermeable material;
forming a sole (236, 336) including a biasing member (241b, 341) biasing the sole (236, 336) out-of-plane along axes perpendicular to its major axis (A1); ;
securing the sole (236, 336) to the foot cuff (10) in generally opposing relationship with the first layer (22) of the inflatable member (14), so that the biasing member (241b, 341) biases the sole (236, 336) in a direction causing a central area of the sole (2236, 336) to move toward the inflatable member (14) and longitudinal ends of the sole (2236, 336) to move away from the inflatable member (14).

## Patentansprüche

1. Kompressionsfußmanschette (10) zum Anlegen einer Kompression an einen Fuß eines Trägers aufweisend:
ein aufblasbares Element (14), das eine erste und zweite flüssigkeitsundurchlässige Lage (22, 24) aufweist, die aneinander befestigt sind, um eine aufblasbare Kammer (27) zu definieren; und
eine Sohle (236, 336), die an die Fußmanschette (10) in generell gegenüberliegender Beziehung zu der ersten Lage (22) des aufblasbaren Elements (14) befestigt ist;
**dadurch gekennzeichnet,**
**dass** die Sohle (236, 336) ein Vorspannelement (241b, 341) aufweist, das die Sohle (236, 336) aus der Ebene heraus entlang Achsen senkrecht zu ihrer Hauptachse (A1) in eine Richtung vorspannt, was einen zentralen Bereich der Sohle (2236, 336) dazu veranlasst, sich hin zu dem aufblasbaren Element (14) zu bewegen, und Längsenden der Sohle (2236, 336) sich weg von dem aufblasbaren Element (14) zu bewegen.

2. Kompressionsfußmanschette (10) wie in Anspruch 1 vorausgesetzt, wobei die Sohle (236) ein biegbares Element (241a) und ein Vorspannelement (241b) aufweist und das Vorspannelement (241b) an dem biegbaren Element (241a) befestigt ist, um es in eine generell konvexe Konfiguration vorzuspannen.

3. Kompressionsfußmanschette (10) wie in Anspruch 2 vorausgesetzt, wobei das Vorspannelement (241b) an einer Außen- oder Innenseite des biegbaren Elements (241a) befestigt ist.

4. Kompressionsfußmanschette (10) wie in Anspruch 2 bis 3 vorausgesetzt,
wobei das biegbare Element (241a) mehrere beabstandete, generell starre Elemente (238) aufweist, die klappbar aneinander entlang der Hauptachse (A2) der Sohle (236) befestigt sind, so dass die Sohle (236) generell aus der Ebene heraus biegbar ist.

5. Kompressionsfußmanschette wie in Anspruch 4 vorausgesetzt, wobei die generell starren Elemente (238) klappbar aneinander durch ein generell biegbares Element (240) befestigt sind.

6. Kompressionsfußmanschette wie in Anspruch 5 vorausgesetzt, wobei die generell starren Elemente (238) eine Dicke aufweisen, die größer als die des generell biegbaren Elements (240) ist.

7. Kompressionsfußmanschette wie in Anspruch 6 vorausgesetzt, wobei die starren Elemente (238) und das generell biegbare Element (240) als ein Materialteil gebildet sind.

8. Kompressionsfußmanschette (10) wie in einem der Ansprüche 5 bis 7 vorausgesetzt, die mehrere generell biegbare Elemente (140) aufweist.

9. Kompressionsfußmanschette (10) wie in Anspruch 1 vorausgesetzt, wobei die Sohle (336) ein Laminat ist, das ein zusammendrückbares Element (343) aufweist und das Vorspannelement (336) an dem zusammendrückbaren Element (343) befestigt ist, um es in die generell konvexe Konfiguration vorzuspannen.

10. Kompressionsfußmanschette (10) wie in Anspruch 9 vorausgesetzt, wobei das Vorspannelement (336) an eine Außen- oder Innenseite des zusammendrückbaren Elements (343) befestigt ist.

11. Verfahren zum Herstellen einer Kompressionsfußmanschette (10) zum Anlegen einer Kompression an einen Fuß eines Trägers, wobei das Verfahren aufweist:
Bilden eines aufblasbaren Elements (14), indem eine erste und zweite Lage (22, 24) aus flüssigkeitsundurchlässigem Material, die sich generell gegenüberliegen, miteinander verbunden werden;
Bilden einer Sohle (236, 336), die ein Vorspannelement (241b, 341) enthält, das die Sohle (236, 336) aus der Ebene heraus entlang Achsen senkrecht zu ihrer Hauptachse (A1) vorspannt;
Befestigen der Sohle (236, 336) an der Fußmanschette (10) in generell gegenüberliegender Beziehung zu der ersten Lage (22) des aufblasbaren Elements (14), so dass das Vorspannelement (241b, 341) die Sohle in eine Richtung vorspannt, was einen zentralen Bereich der Sohle (2236, 336) dazu veranlasst, sich hin zu dem aufblasbaren Element (14) zu bewegen, und Längsenden der Sohle (2236, 336) sich weg von dem aufblasbaren Elements (14) zu bewegen.

## Revendications

1. Collier de pied de compression (10) pour appliquer une compression au pied d'un utilisateur comprenant:
un élément gonflable (14) comportant des première et seconde couches imperméables au fluide (22, 24) fixées l'une à l'autre pour définir une chambre gonflable (27); et
une semelle (236, 336) fixée au collier de pied (10) selon une relation généralement opposée à la première couche (22) de l'élément gonflable (14);
**caractérisé en ce que** la semelle (236, 336) comporte un élément de sollicitation (241b, 341) sollicitant la semelle (236, 336) hors du plan le long d'axes perpendiculaires à son axe majeur (A1) dans une direction amenant une zone centrale de la semelle (2236, 336) à se déplacer vers l'élément gonflable (14) et les extrémités longitudinales de la semelle (2236, 336) à s'éloigner de l'élément gonflable (14).

2. Collier de pied de compression (10) selon la revendication 1, dans lequel la semelle (236) comprend un élément flexible (241a) et un élément de sollicitation (241b), et l'élément de sollicitation (241b) est fixé à l'élément flexible (241a) pour le solliciter dans une configuration généralement convexe.

3. Collier de pied de compression (10) selon la revendication 2, dans lequel l'élément de sollicitation (241b) est fixé à un côté extérieur ou intérieur de l'élément flexible (241a).

4. Collier de pied de compression (10) selon l'une quelconque des revendications 2 à 3, dans lequel l'élément flexible (241a) comporte une pluralité d'éléments espacés, généralement rigides (238) articulés les uns aux autres le long de l'axe majeur (A2) de la semelle (236) de sorte que la semelle (236) peut être courbée généralement hors du plan.

5. Collier de pied de compression selon la revendication 4, dans lequel les éléments généralement rigides (238) sont articulés les uns aux autres par un élément généralement flexible (240).

6. Collier de pied de compression selon la revendication 5, dans lequel les éléments généralement rigides (238) ont des épaisseurs supérieures à l'élément généralement flexible (240).

7. Collier de pied de compression selon la revendication 6, dans lequel les éléments rigides (238) et l'élément généralement flexible (240) sont réalisés comme une pièce de matériau.

8. Collier de pied de compression (10) selon l'une quelconque des revendications 5 à 7, comprenant une pluralité d'éléments généralement flexibles (140).

9. Collier de pied de compression (10) selon la revendication 1, dans lequel la semelle (336) est un laminé incluant un élément compressible (343), et l'élément de sollicitation (336) est fixé à l'élément compressible (343) pour le solliciter en une configuration généralement convexe.

10. Collier de pied de compression (10) selon la revendication 9, dans lequel l'élément de sollicitation (336) est fixé à un côté extérieur ou intérieur de l'élément compressible (343).

11. Procédé de fabrication d'un collier de pied de compression (10) pour appliquer une compression au pied d'un utilisateur, le procédé comprenant:
former un élément gonflable (14) en reliant ensemble des première et seconde couches (22, 24) généralement opposées en un matériau imperméable au fluide;
former une semelle (236, 336) incluant un élément de sollicitation (241b, 341) sollicitant la semelle (236, 336) hors du plan le long d'axes perpendiculaires à son axe majeur (A1);
fixer la semelle (236, 336) au collier de pied (10) selon une relation généralement opposée avec la première couche (22) de l'élément gonflable (14) de sorte que l'élément de sollicitation (241b, 341) sollicite la semelle (236, 336) dans une direction amenant une zone centrale de la semelle (2236, 336) à se déplacer vers l'élément gonflable (14) et les extrémités longitudinales de la semelle (2236, 336) à s'éloigner de l'élément gonflable (14).
